# EUROPEAN PATENT SPECIFICATION

(11) **EP 4 155 379 B1**
(45) Date of publication and mention of the grant of the patent: **20.03.2024**
(21) Application number: 21199594.9
(22) Date of filing: 28.09.2021
(51) Int. Cl.: C12M 1/00, C12M 3/00, C12M 1/34

(54) **SMART BIOPROCESS TUBE LINE ASSEMBLY**
INTELLIGENTE ROHRLEITUNGSANORDNUNG FÜR BIOPROZESS
ENSEMBLE LIGNE DE TUBE INTELLIGENT POUR BIOPROCÉDÉ

(43) Date of publication of application: 29.03.2023
(73) Proprietor: Sartorius Stedim Biotech GmbH, 37079 Göttingen (DE)
(72) Inventor: Grimm, Christian, 37079 Göttingen (DE)
(74) Representative: Vigand, Philippe

(56) References cited:
- US-A1- 2012 206 155
- US-A1- 2018 252 341
- US-A1- 2020 108 242

## Description

The invention relates to a smart tube line assembly for use in a unit operation of a bioprocess. The invention further relates to a method of using such a smart tube line assembly.

In many bioprocess unit operations which are based on single-use (disposable) equipment, disposable tube line assemblies play an important role in today's product development and commercial manufacturing. One of the main challenges in setting up a unit operation using a reusable skid is to connect and apply the tubing, including sensors, actuators and sterile/aseptic connectors to the respective holders or connecting elements at the skid. In addition, there is a fundamental need of having a full integrity of the tube line assembly during process, to ensure operator and process safety. Furthermore, sensing elements and actuator embodiments are located in a certain physical distance from the central control unit or the skid, which includes the central control unit, to transfer electrical power and/or data between sensors or actuators and the control unit.

The current way of setting up and installing a tube line assembly to a skid is a manual process supported by written instructions and some fixing elements at the skid to reduce human error. The correct connection of the sterile/aseptic connectors to each other is only checked by visual inspection of the operator. The integrity / leak tightness of a single-use tube line assembly is only tested before and in some cases after use of the assembly, but not during operation. Sensors and actuators are connected via separate cables to power and data interfaces, or the data transmission is established by a wireless connection in few cases. Especially in high pressure applications there is a need to use reinforced tubes, which lead to certain opaqueness of the tubes and a very limited ability to see if fluid is present or flowing through the tubes.

US 2020/0255787 A1 shows a container for the growth of biological entities that is illuminated by a flexible light diffusing fiber. The light diffusing fiber includes a core formed from a silica-based glass and a cladding in direct contact with the core. The light diffusing fiber also includes an outer polymer coating layer surrounding the cladding, the outer polymer coating layer being the cured product of a liquid polymer blend including a scattering material and a luminophore.

US 2012/0206155 A1 discloses a system for measuring parameters in a container, which can have a liquid flow or no liquid flow. The system includes a tag and a sensor on the tag incorporated or integrated into the container. The container can be a bag, a tube, a pipe, or a hose. This prior art document discloses a silicone tubing 1000 with differing diameters that produce differential pressure as fluid flows through it. According to a specific embodiment, a silicone tubing can be embedded with RFID pressure sensors.

US 2018/0252341 A1 shows an endoscopic tube having a tube body in which high-pressure and low-pressure lumina are embedded as a lumen bundle. A metal strand or fiber, a polymer fiber, or an optical fiber may be embedded the tube body for signal transmission, absorption of tensile forces, or guiding light.

The object of the present invention is to overcome the above-mentioned limitations in handling and use of single-use tube line assemblies in bioprocess unit operations.

The above problem is solved by a tube line assembly according to claim 1. Advantageous and expedient embodiments of the invention are apparent from the dependent claims.

The invention provides a smart single-use tube line assembly for use in a unit operation of a bioprocess. The tube line assembly comprises at least one tube line. The tube line includes a tube body surrounding a lumen through which a medium can flow. The tube line assembly also comprises a functional element embedded in the tube body, the functional element being configured to perform an optical and/or electrical function. The tube line assembly further comprises an interface for coupling both the lumen and the functional element of the tube line to a matching interface of another component allowing liquid medium to be transferred from the tube line to the other component and for connecting the functional element, respectively, at the same time. The tube line assembly still further comprises a control unit for controlling the optical and/or electrical function.

The invention is based on the finding that a tube line assembly can be modified and made smart to determine or gather information about the status of the bioprocess system setup, especially the tube line assembly, or of the bioprocess itself. This can be accomplished through a sensor function (correct process conditions, safe setup state, correct and safe connections etc.) or through associated signal lines for the corresponding sensor data. Furthermore, the status can not only be detected, but can also be actively visualized, e.g. via lighting elements such as LEDs, optical fibers etc. The integrated functional elements of the tube line assembly according to the invention can support the operator setting up and installing bioprocess equipment to be used for performing a unit operation and to help the operator to follow the running process. Correct connections can be indicated as well as incorrect connections, and detected failures can trigger an alarm or a safety shut-off. Accordingly, the risk of human errors and integrity issues can be significantly reduced.

According to a preferred embodiment of the tube line assembly, the tube body and the functional element are coextruded. It was found that it is possible to incorporate most of the typical materials a functional element in the sense of the present invention is made of when the tube body is produced in an extrusion process, thus allowing a simplified manufacturing of the tube line assembly. Compared to known methods of embedding a functional element, coextruding of a tube body with, for example, an optical fiber made of a material like polycarbonate (PC), polymethyl methacrylate (PMMA), glass etc. is a very efficient and reliable manufacturing process.

In case the functional element embedded in the tube body of the tube line assembly is used to perform an electrical function, the functional element preferably includes one or more conductor elements like metallic layers or sheets or wires. Such conductor elements can be used for various purposes. In particular, a conductor element can form at least one of the following: a power supply line for transmitting electrical power; a signal line or for transmitting electrical signals or data; a sensor element for electrical resistance and/or capacitance measurements. In general, the embedded conductor elements help to save cables and - due to the routing given by the course of the tube line - to prevent wrong cable routing in the setup. Electrical resistance and/or capacitance measurements made with the conductor elements can be used to confirm integrity or to detect physical damages of a tube line or a connection.

According to a special variant, the conductor element(s) of the functional element helically surround(s) the lumen to reinforce the tube body. Especially when electrical wires are used as conductor elements they can perform the additional function of providing extra mechanical strength to the tube body.

The conductor element(s) of the functional element, especially electrical wires, can be surrounded by a shielding layer to reduce electromagnetic interference.

In case the functional element embedded in the tube body of the tube line assembly is used to perform an optical function, especially a visualization function, the functional element can include one or more optical fibers for transmitting light or illuminating the tube line by decoupling light from an optical fiber over its full length or over certain portions. The transmitted light or the illumination can be used to indicate a flow (direction) of a medium inside the tube line or to indicate a correct connection of the tube line, for example.

For illumination purposes the functional element can also include a plurality of light sources, preferably LEDs arranged on a strip. The light sources are embedded in the tube body in such a manner that the emitted light is visible under regular operating conditions.

As already mentioned, the functional element can be used to determine certain conditions of the bioprocess system setup, especially the tube line assembly, or of the bioprocess itself. This means that the functional element is not only used for powering a sensor element or transferring signals or data from/to one or more sensor elements, but that the functional element itself constitutes one or more sensor elements. In particular, such a sensor element can detect one or more of the following parameters: temperature, flow, pressure, mechanical stress, moisture.

According to a special aspect of the invention, one or more of the sensor elements include a fiber Bragg grating. The optical properties of the fiber Bragg grating depend on the current mechanical conditions of the surrounding tube body. Therefore, the embedded fiber Bragg grating can be used as a sensor element for detecting at least one of the following: bending or kinking of the tube line; pressure in the tube line; change in wall/layer thickness of the tube line; leakage.

In order to couple both the lumen and the functional element of the tube line to another component via a sterile or aseptic connector, such a connector should include an interface matching the interface of the tube line.

Depending on the kind and optical and/or electrical functionality of the functional element, the control unit of the tube line assembly is configured accordingly to control input of light and/or electrical power and/or electrical signals and/or data into the functional element.

The invention also provides a method of using a smart single-use tube line assembly as described above during setup and/or operation of a bioprocess.

Preferably, the functional element and the control unit are used to optically and/or electrically indicate at least one of the following:
- completed connection of the hose line;
- correct connection of the hose line;
- presence of a liquid medium in the tube line;
- flow, and preferably flow direction, of a liquid medium in the tube line;
- a severe condition in the tube line like overpressure, overheating, mechanical stress, leakage etc.

Further features and advantages of the invention will become apparent from the following description and from the accompanying drawings to which reference is made. In the drawings:
- Figure 1 shows a sectional view of a part of a first embodiment of the tube line assembly according to the invention;
- Figure 2 shows a perspective view of a part of a second embodiment of the tube line assembly according to the invention;
- Figure 3 shows a sectional view of a part of a third embodiment of the tube line assembly according to the invention;
- Figure 4 shows a side view of the connector of the tube line assembly of Figure 3; and
- Figure 5 shows a perspective view of one end of the connector of Figure 4.

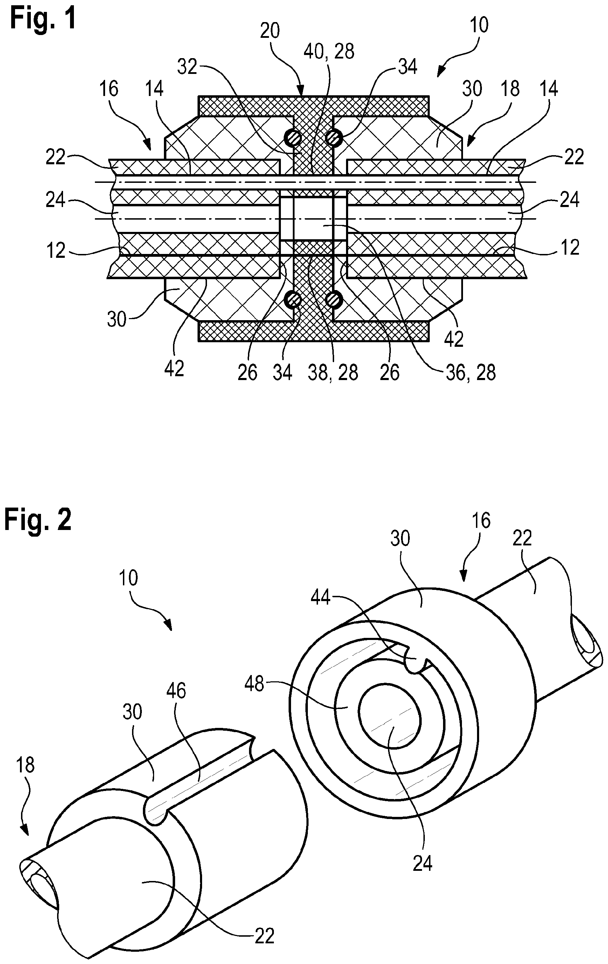

Figures 1 shows one example of a tube line assembly 10 incorporating functional elements 12, 14. One end of a first tube line 16 and one end of a second tube line 18 are coupled together via an aseptic or sterile connector 20.

Each tube line 16, 18 includes a tube body 22 surrounding a lumen 24 through which a liquid medium can flow. Embedded in the tube bodies 22 are two functional elements 12, 14. The first functional element 12 is a conductor element in the form of a wire or a cable. The second functional element 14 is an optical fiber.

The functional elements 12, 14 can be designed differently and/or include further elements and can be used for any of the purposes as described in the general portion of the description.

Both tube line ends include an interface 26 for coupling the lumen 24 and each functional element 12, 14 of the respective tube line 16, 18 to a matching interface 28 of the connector 20. Here, the tube line interfaces 26 are formed in rigid end pieces 30 provided at the tube line ends, and the connector interface 28 is provided in a middle wall section 32 of the connector 20. For sealing purposes, O-rings 34 are provided between the end pieces 30 and the middle wall section 32 of the connector 20.

In the particular embodiment shown in Figure 1 the connector interface 28 includes a central through opening 36 with a diameter corresponding to the diameter of the lumina 24 of the tube lines 16, 18. The connector interface 28 further includes electrical and optical connecting elements 38, 40 corresponding to the functional elements 12, 14 of the tube lines 16, 18, respectively.

For coupling both the lumina 24 and the functional elements 12, 14 of the two tube lines 16, 18 together, the tube line ends are inserted into opposite receptacles 42 of the connector 20 which are separated from each other by the middle wall section 32. Alignment means (examples of which will be described in connection with the other embodiments) ensure that the interfaces 26, 28 of the tube lines 16, 18 and the connector 20 are correctly aligned so that the ends of the corresponding functional elements 12, 14 face each other.

In the coupled state, as shown in Figure 1, liquid medium, light and electrical current can be conducted from one tube line 14 to the other tube line 16, or vice versa, through the connector 20 via the interfaces 26, 28. The coupling of the functional elements 12, 14 can be used to indicate a correct and successful connection in the setup and/or for other purposes as described before. The corresponding electrical and optical functions are controlled by a control unit (not shown) of the tube line assembly 10.

The connector 20 can also be used to connect one tube line 16, 18 to a component other than a tube line 16, 18, e.g. a vessel, a pump etc. The interface 26 of a tube line 16, 18 can also be used to directly connect the lumen 24 of the tube line 16, 18 and its functional element(s) 12, 14 to a port of another component without a connector 20. In such a case the port of the other component includes an interface matching the interface 26 of the tube line 16, 18.

Figure 2 shows a variant of the above-described example which allows coupling of two tube lines 16, 18 without a separate connector 20. Here, the end pieces 30 of the tube lines 16, 18 are configured such that the end piece 30 of the second tube line 18 can be inserted into the end piece 30 of the first tube line 16. A nose 44 on the end piece 30 of the first tube line 16 corresponds with a groove 46 formed in the end piece 30 of the second tube line 18. Accordingly, the end pieces 30 can only be connected in the correct orientation with respect to the functional elements 12, 14 (not explicitly shown here). Of course, the nose/groove arrangement can be vice versa, or other alignment means can be used.

The end piece 30 of the second tube line 18 is inserted into the end piece 30 of the first tube line 16 until contact faces 48 of the tube line ends contact each other. Additional sealing means may be provided. In this coupled state of the tube lines 16, 18, the matching interfaces 26 of the tube lines 16, 18 ensure that liquid medium, light and electrical current can be transferred from one tube line 16 to the other tube line 18, or vice versa, through the lumina 24 and the functional elements 12, 14, respectively.

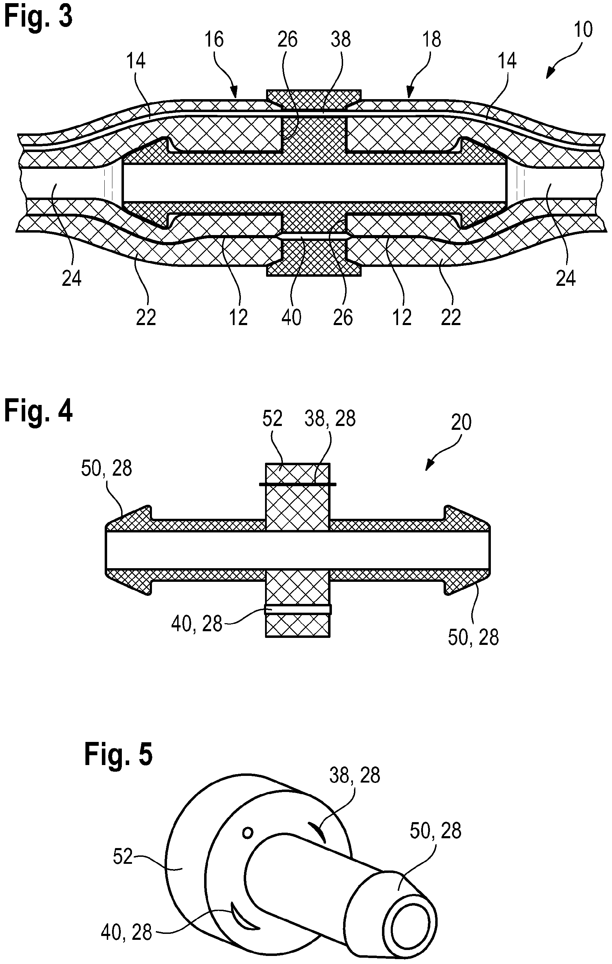

Figures 3 to 5 show a further embodiment of the tube line assembly 10 with a generally cross shaped connector 20. The connector 20, which is shown separately in Figure 3, has two opposite hose barb fittings 50 and a middle portion 52 extending perpendicular from the hose barb fittings 50. While the hose barb fittings 50 constitute a flow interface allowing a liquid medium to flow through the connector 20, the middle portion 52 is provided with electrical and optical interfaces including electrical and optical connecting elements 38, 40 for connecting the functional elements 12, 14.

The tube line ends are pushed over the hose barb fittings 50 until they contact the middle portion 52 of the connector 20, as shown in Figure 4. Alignment means, such as corresponding markings or interacting mechanical structures on the tube line ends and on the connector 20, ensure that the tube lines 16, 18 are in the correct orientation so that the electrical and optical interfaces of the tube lines 16, 18 match with those of the connector 20.

In all embodiments the tube lines 16, 18 and the connector 20 (if present) and their sub-components are made of single-use materials, i.e. plastic materials that can be sterilized, especially with gamma radiation, without significantly impairing their mechanical properties (stability, brittleness etc.).

It is to be understood that the features of the embodiments described above can be combined in a suitable manner.

### List of Reference Signs

10 tube line assembly
12 (electrical) functional element
14 (optical) functional element
16 first tube line
18 second tube line
20 connector
22 tube body
24 lumen
26 tube line interface
28 connector interface
30 end piece
32 wall section
34 O-rings
36 opening
38 electrical connecting element
40 optical connecting element
42 receptacles
44 nose
46 groove
48 contact face
50 hose barb fittings
52 middle portion

## Claims

1. A smart single-use tube line assembly (10) for use in a unit operation of a bioprocess, comprising
at least one tube line (16, 18), the tube line (16, 18) including a tube body (22) surrounding a lumen (24) through which a medium can flow, and
a functional element (12, 14) embedded in the tube body (22), the functional element (12, 14) being configured to perform an optical and/or electrical function,
**characterized by**
an interface (26) for coupling both the lumen (24) and the functional element (12, 14) of the tube line (16, 18) to a matching interface of another component, allowing liquid medium to be transferred from the tube line (16, 18) to the other component and for connecting the functional element (12, 14), respectively, at the same time, and
a control unit for controlling the optical and/or electrical function.

2. The tube line assembly (10) according to claim 1, **characterized in that** the tube body (22) and the functional element (12, 14) are coextruded.

3. The tube line assembly (10) according to claim 1 or 2, **characterized in that** the functional element (12) includes one or more conductor elements forming
a power supply line for transmitting electrical power, and/or
a signal line or for transmitting electrical signals or data, and/or
a sensor element.

4. The tube line assembly (10) according to claim 3, **characterized in that** the conductor element helically surrounds the lumen (24) to reinforce the tube body (22).

5. The tube line assembly (10) according to claim 3 or 4, **characterized in that** the conductor element is surrounded by a shielding layer.

6. The tube line assembly (10) according to any of the preceding claims, **characterized in that** the functional element (14) includes one or more optical fibers for transmitting light or illuminating the tube line (16, 18).

7. The tube line assembly (10) according to any of the preceding claims, **characterized in that** the functional element (14) includes a plurality of light sources, preferably LEDs arranged on a strip.

8. The tube line assembly (10) according to any of the preceding claims, **characterized in that** the functional element (12, 14) includes one or more sensor elements, preferably detecting one or more of the following parameters: temperature, flow, pressure, mechanical stress, moisture.

9. The tube line assembly (10) according to claim 8, **characterized in that** one of the sensor elements includes a fiber Bragg grating.

10. The tube line assembly (10) according to any of the preceding claims, **characterized by** a sterile or aseptic connector (20) including an interface (28) matching the interface (26) of the tube line (16, 18).

11. The tube line assembly (10) according to any of the preceding claims, **characterized in that** the control unit is configured to control input of light and/or electrical power and/or electrical signals and/or data into the functional element (12, 14).

12. A method of using a smart single-use tube line assembly (10) according to one of the preceding claims during setup and/or operation of a bioprocess.

13. The method according to claim 12, **characterized in that** the functional element (12, 14) and the control unit are used to optically and/or electrically indicate at least one of the following:
- completed connection of the hose line;
- correct connection of the hose line;
- presence of a liquid medium in the tube line (16, 18);
- flow, and preferably flow direction, of a liquid medium in the tube line (16, 18);
- a severe condition in the tube line (16, 18).

## Patentansprüche

1. Intelligente Rohrleitungsanordnung (10) zur einmaligen Verwendung bei einer Grundoperation eines Bioprozesses, die Folgendes umfasst
mindestens eine Rohrleitung (16, 18), wobei die Rohrleitung (16, 18) einen Rohrkörper (22) beinhaltet, der ein Lumen (24) umgibt, durch das ein Medium fließen kann, und
ein Funktionselement (12, 14), das in den Rohrkörper (22) eingebettet ist, wobei das Funktionselement (12, 14) dazu ausgelegt ist, eine optische und/oder eine elektrische Funktion durchzuführen,
**gekennzeichnet durch**
eine Schnittstelle (26) zum Koppeln sowohl des Lumens (24) als auch des Funktionselements (12, 14) der Rohrleitung (16, 18) an eine passende Schnittstelle einer anderen Komponente, wodurch es erlaubt wird, dass ein flüssiges Medium von der Rohrleitung (16, 18) zur anderen Komponente transportiert wird, bzw. zum gleichzeitigen Verbinden des Funktionselements (12, 14), und
eine Steuereinheit zum Steuern der optischen und/oder der elektrischen Funktion.

2. Rohrleitungsanordnung (10) nach Anspruch 1, **dadurch gekennzeichnet, dass** der Rohrkörper (22) und das Funktionselement (12, 14) coextrudiert sind.

3. Rohrleitungsanordnung (10) nach Anspruch 1 oder 2, **dadurch gekennzeichnet, dass** das Funktionselement (12) ein oder mehrere Leiterelemente beinhaltet, die Folgendes bilden
eine Stromversorgungsleitung zum Übertragen von elektrischem Strom, und/oder
eine Signalleitung zum Übertragen von elektrischen Signalen oder Daten, und/oder
ein Sensorelement.

4. Rohrleitungsanordnung (10) nach Anspruch 3, **dadurch gekennzeichnet, dass** das Leiterelement das Lumen (24) schraubenförmig umgibt, um den Rohrkörper (22) zu verstärken.

5. Rohrleitungsanordnung (10) nach Anspruch 3 oder 4, **dadurch gekennzeichnet, dass** das Leiterelement von einer Schirmungsschicht umgeben ist.

6. Rohrleitungsanordnung (10) nach einem der vorhergehenden Ansprüche, **dadurch gekennzeichnet, dass** das Funktionselement (14) zum Übertragen von Licht oder zum Beleuchten der Rohrleitung (16, 18) eine oder mehrere optische Fasern beinhaltet.

7. Rohrleitungsanordnung (10) nach einem der vorhergehenden Ansprüche, **dadurch gekennzeichnet, dass** das Funktionselement (14) eine Vielzahl von Lichtquellen beinhaltet, vorzugsweise LEDs, die auf einem Streifen angeordnet sind.

8. Rohrleitungsanordnung (10) nach einem der vorhergehenden Ansprüche, **dadurch gekennzeichnet, dass** das Funktionselement (12, 14) ein oder mehrere Sensorelemente beinhaltet, die vorzugsweise einen oder mehrere der folgenden Parameter detektieren: Temperatur, Fluss, Druck, mechanische Beanspruchung, Feuchtigkeit.

9. Rohrleitungsanordnung (10) nach Anspruch 8, **dadurch gekennzeichnet, dass** eines der Sensorelemente ein Bragg-Fasergitter beinhaltet.

10. Rohrleitungsanordnung (10) nach einem der vorhergehenden Ansprüche, die durch einen sterilen oder aseptischen Verbinder (20) gekennzeichnet ist, der eine Schnittstelle (28) beinhaltet, die zur Schnittstelle (26) der Rohrleitung (16, 18) passt.

11. Rohrleitungsanordnung (10) nach einem der vorhergehenden Ansprüche, **dadurch gekennzeichnet, dass** die Steuereinheit dazu ausgelegt ist, eine Eingabe von Licht und/oder elektrischem Strom und/oder elektrischen Signalen und/oder Daten in das Funktionselement (12, 14) zu steuern.

12. Verfahren zum Verwenden einer intelligenten Rohrleitungsanordnung (10) zur einmaligen Verwendung nach einem der vorhergehenden Ansprüche während einer Einrichtung und/oder eines Betriebs eines Bioprozesses.

13. Verfahren nach Anspruch 12, **dadurch gekennzeichnet, dass** das Funktionselement (12, 14) und die Steuereinheit verwendet werden, um eines von Folgendem optisch und/oder elektrisch anzuzeigen:
- abgeschlossene Verbindung der Schlauchleitung;
- korrekte Verbindung der Schlauchleitung;
- Vorhandensein eines flüssigen Mediums in der Rohrleitung (16, 18);
- Fluss, und vorzugsweise eine Flussrichtung, eines flüssigen Mediums in der Rohrleitung (16, 18);
- einen schweren Zustand in der Rohrleitung (16, 18).

## Revendications

1. Ensemble de conduite tubulaire intelligent à usage unique (10) destiné à être utilisé dans une opération unitaire d'un bioprocédé, comprenant :
au moins une conduite tubulaire (16, 18), la conduite tubulaire (16, 18) comprenant un corps de tube (22) entourant une lumière (24) à travers laquelle peut s'écouler un milieu, et
un élément fonctionnel (12, 14) encastré dans le corps de tube (22), l'élément fonctionnel (12, 14) étant configuré pour réaliser une fonction optique et/ou électrique,
**caractérisé par** :
une interface (26) pour coupler à la fois la lumière (24) et l'élément fonctionnel (12, 14) de la conduite tubulaire (16, 18) à une interface correspondante d'un autre composant, permettant au milieu liquide d'être transféré de la conduite tubulaire (16, 18) à l'autre composant et pour raccorder l'élément fonctionnel (12, 14), respectivement en même temps, et
une unité de commande pour commander la fonction optique et/ou électrique.

2. Ensemble de conduite tubulaire (10) selon la revendication 1, **caractérisé en ce que** le corps de tube (22) et l'élément fonctionnel (12, 14) sont coextrudés.

3. Ensemble de conduite tubulaire (10) selon la revendication 1 ou 2, **caractérisé en ce que** l'élément fonctionnel (12) comprend un ou plusieurs éléments conducteurs formant :
une ligne d'alimentation électrique pour transmettre l'énergie électrique, et/ou
une ligne de signal pour transmettre des signaux électriques ou des données, et/ou
un élément de capteur.

4. Ensemble de conduite tubulaire (10) selon la revendication 3, **caractérisé en ce que** l'élément conducteur entoure, de manière hélicoïdale, la lumière (24) afin de renforcer le corps de tube (22).

5. Ensemble de conduite tubulaire (10) selon la revendication 3 ou 4, **caractérisé en ce que** l'élément conducteur est entouré par une couche de protection.

6. Ensemble de conduite tubulaire (10) selon l'une quelconque des revendications précédentes, **caractérisé en ce que** l'élément fonctionnel (14) comprend une ou plusieurs fibres optiques pour transmettre la lumière ou éclairer la conduite tubulaire (16, 18).

7. Ensemble de conduite tubulaire (10) selon l'une quelconque des revendications précédentes, **caractérisé en ce que** l'élément fonctionnel (14) comprend une pluralité de sources lumineuses, de préférence des LED agencées sur une bande.

8. Ensemble de conduite tubulaire (10) selon l'une quelconque des revendications précédentes, **caractérisé en ce que** l'élément fonctionnel (12, 14) comprend un ou plusieurs éléments de capteur, de préférence détectant un ou plusieurs des paramètres suivants : la température, le flux, la pression, la contrainte mécanique, l'humidité.

9. Ensemble de conduite tubulaire (10) selon la revendication 8, **caractérisé en ce que** l'un des éléments de capteur comprend un réseau de Bragg sur fibre.

10. Ensemble de conduite tubulaire (10) selon l'une quelconque des revendications précédentes, **caractérisé par** un connecteur stérile ou aseptique (20) comprenant une interface (28) correspondant à l'interface (26) de la conduite tubulaire (16, 18).

11. Ensemble de conduite tubulaire (10) selon l'une quelconque des revendications précédentes, **caractérisé en ce que** l'unité de commande est configurée pour commander l'entrée de lumière et/ou d'énergie électrique et/ou de signaux électriques et/ou de données dans l'élément fonctionnel (12, 14).

12. Procédé pour utiliser un ensemble de conduite tubulaire intelligent à usage unique (10) selon l'une des revendications précédentes pendant l'installation et/ou le fonctionnement d'un bioprocédé.

13. Procédé selon la revendication 12, **caractérisé en ce que** l'élément fonctionnel (12, 14) et l'unité de commande sont utilisés pour indiquer par voie optique et/ou par voie électrique au moins l'un des éléments suivants :
le raccordement terminé de la conduite flexible ;
le raccordement correct de la conduite flexible ;
la présence d'un milieu liquide dans la conduite tubulaire (16, 18) ;
le flux et de préférence la direction de flux d'un milieu liquide dans la conduite tubulaire (16, 18) ;
une condition grave dans la conduite tubulaire (16, 18).
